# EUROPEAN PATENT SPECIFICATION

(11) **EP 0 728 445 B1**
(45) Date of publication and mention of the grant of the patent: **22.12.1999**
(21) Application number: 96301124.2
(22) Date of filing: 20.02.1996
(51) Int. Cl.: A61B 17/06

(54) **Suture package**
Nahtmaterialverpackung
Emballage de suture

(30) Priority: 21.02.1995 US 391890
(43) Date of publication of application: 28.08.1996
(73) Proprietor: ETHICON, INC., Somerville, N J 08876 (US)
(72) Inventor: Siernos, Joseph, Whitehouse Station, New Jersey 08899 (US); Szabo, David A., Branchburg, New Jersey 08876 (US)
(74) Representative: Fisher, Adrian John

(56) References cited:
- US-A- 5 222 978

## Description

The present invention relates to suture packages and, more particularly, the present invention relates to single piece packages for retaining a needle and suture combination in a manner wherein it may be readily dispensed.

### BACKGROUND OF THE INVENTION

Numerous types and styles of suture packages are known in the art. The goal of most of these packages is to present a needle and suture combination to the surgeon in a manner that is consistently convenient and unobtrusive, so that the surgical procedure is uninterrupted when a particular needle and suture combination is requested.

In order to be useful in the operating room, suture packages must be constructed so that they can be handled both before and after they are placed in the sterile field. To this end, current packages are actually a combination of packages and packaging materials. For example, the actual suture-containing package is frequently placed inside an outer envelope or pouch that is sometimes made of metal foil. The outside packaging can be grasped and pulled apart, permitting the sterile package and its contents to be dropped on to a tray within the sterile field.

The sterile package containing the needle and suture may in turn be contained within a paper sleeve or the like to ensure the retention of the needle and suture. Additionally, this outer wrapper serves as a vehicle for marking sizes, dates, types and other pertinent information, including color-coding. Inside this outer wrapper is the actual suture holder, which typically contains a needle swaged to a suture. The needle is held in a predetermined position in a needle park, and the suture material is preferably arrayed and retained within the package so that it is not likely to kink, twist or knot when it is dispensed. Ideally, the suture holder is designed so that the needle is easily grasped by a needle holder, removed, and put into use with as little wasted motion as possible.

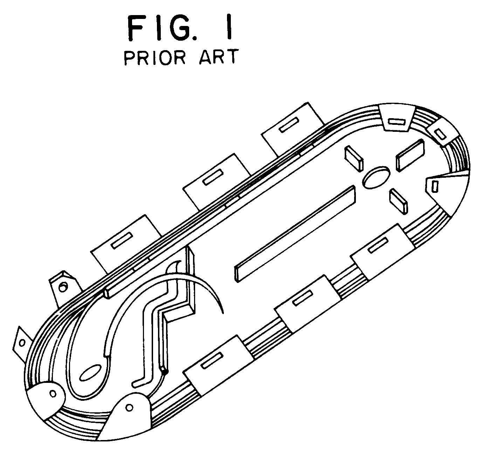

One current design of a suture package is disclosed in U.S. Patent 4,967,902--Sobel et al., which is assigned to the assignee of the present invention. The Sobel patent discloses and claims a one-pieced channel package, as seen in FIG. 1 herein, that comprises a plastic carrier and a plurality of peripheral "doors" that are hinged and can be locked over a peripheral channel in which the suture material is wound. A molded needle park is located in the interior of the package, and a needle is snapped into position and held by molded projections that form the needle park structure.

Although the design disclosed by the Sobel patent presents a package that meets the requirements set forth above, it is also desirable to meet these requirements in manner that reduces the cost and increases the efficiency of the manufacturing process. Suture packages are manufactured in high volumes under the strictest standards of quality control. It is therefore important to provide a package that can be manufactured using a process that can be reliably practiced at high volume. The goal is thus to provide simple packages that are easily filled and inspected. It is therefore an object of the present invention to provide a suture package that is easily filled using automated winding equipment.

Another consideration in the manufacture of packaging for certain types of sutures is the effects of humidity upon the suture material. In particular, absorbable sutures are not tolerant of high humidity and degrade. As understood by those of skill in the art, providing a hermetically sealed package is neither easy nor economical. This problem is addressed by the package disclosed in U.S. Patent No. 5,222,978--Kaplan et al the disclosure of which forms the basis for the preamble of claim 1. Kaplan et al teaches filling absorbable sutures with a stabilizing agent and placing them in a sealed package that has a narrow convoluted passageway. The package containing the suture is then placed in a foil pouch along with a package stabilizing element, such as a paper sheet filled with a stabilizing element. The resulting package must then be equilibrated at a relatively high humidity level.

Thus, one solution is to provide a desiccant material within the package. However, the placement of a desiccant in the package often interferes with the dispensing of the suture or needle, or both, and thus, as discussed above, interferes with the surgical procedure. It is therefore also an object of the present invention to provide a package that can readily accept a desiccant material that does not interfere with surgical procedures or require any additional steps when the package is presented to the needle holder.

Finally, as explained above, it is desirable, and in fact extremely important, to provide a mechanism by which the suture material can be dispensed (unwound) from the package efficiently and without twisting or forming kinks or knots. Ideally, the suture should extend to its full unwound length as soon as the needle is placed in the needle holder. It is therefore an additional object of the present invention to provide a package whereby the suture material falls freely away from the package as a natural result of the needle holder loading operation.

### SUMMARY OF THE INVENTION

According to the present invention there is disclosed a package for retaining suture material as defined in claim 1. The package retains a sheet of desiccant material that preferably has a tendency to absorb moisture more readily than the suture material. The package may be rotary wound from the bottom and the suture is dispensed either by pulling the suture material through a central dispensing port or by pushing out the desiccant sheet using the needle holder, so the suture material falls away from the package and uncoils. The latter dispensing method is known as "self-dispensing" and will preferably occur as a natural result of the needle holder pressing against a flap located in the vicinity of the needle park.

### BRIEF DESCRIPTION OF THE DRAWINGS

FIG. 1 is a perspective view of a suture package made in accordance with the prior art;
FIG. 2 is a plan view of a suture package made in accordance with the present invention;
FIG. 3 is a cross-sectional view of a suture package made in accordance with the present invention taken along lines 3-3 in FIG. 1;
FIG. 4 is a plan view, similar to FIG. 2, of an alternate embodiment of a suture package made in accordance with the present invention;
FIG. 5 is a side elevation view, similar to FIG. 4, of the suture package shown in FIG. 4.
FIG. 6 is a plan view of the suture package shown in FIG. 4, loaded with a suture and needle; and
FIG. 7 is an exploded perspective view of the loaded suture package shown in FIG. 6, illustrating the placement of a sheet of desiccant material.

### DETAILED DESCRIPTION OF THE PREFERRED EMBODIMENTS

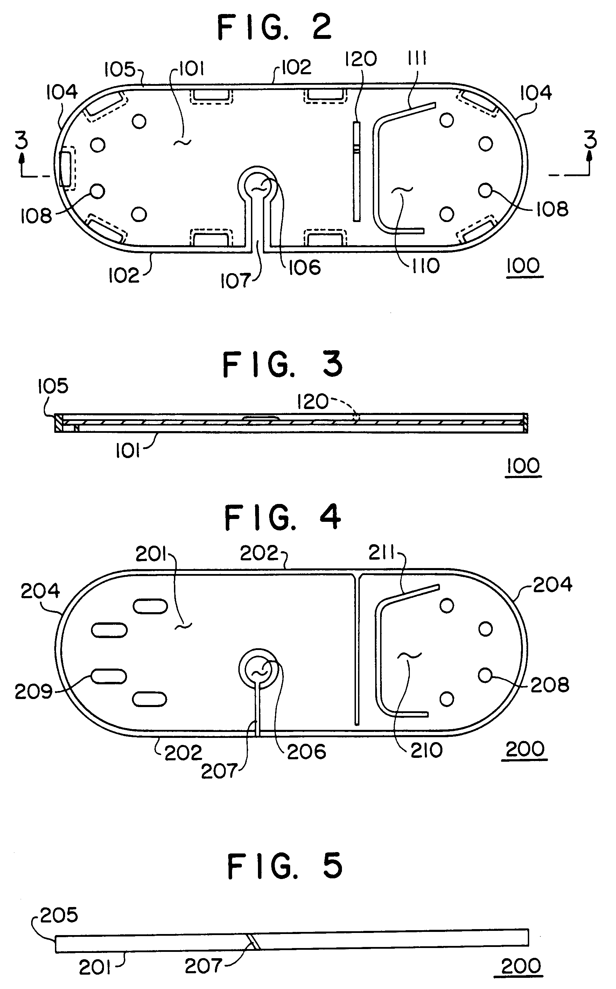

Referring now to FIGS. 2-3, there is shown, respectively, a top plan view and cross-sectional view of a suture holder 100 made in accordance with the present invention. Preferably, the structure shown is molded as a single piece of plastic. An FDA approved and recyclable plastic are most preferable, the former being a mandatory requirement. Because the present invention does not require slip agents, mold release elements or the like, molding costs are reduced. However, it will be appreciated that the suture holder 100 can also be machined from a solid sheet of plastic, thermoformed, or formed by other techniques. Additionally, a composite structure formed by "outsert" molding structural plastic components on to a sheet of another material, e.g., paper may also utilize the advantages of the present invention disclosed herein. Thus, the present invention is not limited to any particular manufacturing technique, nor to plastics; however, molded plastic represents a preferred embodiment.

As seen in FIG. 2, the suture package 100 is most preferably of a shape having parallel sides 102 and rounded ends 104. The straight sides 102 permit the package to be slipped into a protective sleeve, if desired, and the rounded ends 104 provide an appropriate geometry for the wound suture material, conserving space without introducing a severe bend or radius that could kink the suture material. Of course, in alternate embodiments, numerous other shapes such as circular, rectangular or oval packages may be used. One feature of the suture package that is visible in FIG. 2 is the central dispensing port 106, which preferably is extended outwardly to the periphery of the package 100 by a slot 107, as shown. As explained below, in certain embodiments the slot 107 will be relatively narrow in order to act as a suture end retention location.

Also visible in FIG. 2 are the winding holes 108 that are used when the package 100 is filled. As explained below, the package 100 is placed atop a set of vertically upstanding pins that are in registry with the holes 108. Suture material is then easily wound around the pins to create a spiral winding. As known in the art, this operation can be automated or partially automated, so that the packages 100 can be filled efficiently.

Another feature of the present invention is the provision of a flexible flap 110, preferably formed by cutting a narrow slot 111 in the "floor" 101 of the package 100. The flexible flap 110 is located in the vicinity of the needle park 120 and, as explained below, the flexible flap 110 can be displaced by the needle holder when the package is in use so that the sheet of desiccant material forming the backing structure that is in place to retain the suture material in place is dislodged. This permits the suture material to fall freely from the package, i.e., creates a package that is "self-dispensing."

Finally, also visible in the in the embodiment illustrated in FIG. 2 are a series of peripheral openings 112 with surrounding raised walls 113. These structures, although shown in this embodiment, are optional and aid in the retention of the suture and permit the package to "breathe" so that the desiccant backing describe below absorbs any moisture in the vicinity of the suture material .

The cross-sectional view of FIG. 3 illustrates the "two-sided" nature of the suture package 100 made in accordance with the present invention. As shown, the package 100 comprises a substantially flat floor 101 surrounded by a raised flange 105 that preferably extends around the entire periphery and rises above both sides of the floor 101. Also seen in FIGS. 2-3 is the structure of one embodiment of a needle park 120 useful with the present invention. Although illustrated as a ridge rising above the floor, it will be understood that numerous alternate needle park structures can either be integrally molded with the package 100 or attached to the package, e.g., a block of foam into which the needle are stuck, or drops of releasable adhesive that capture the needle body. Thus, the present invention is not intended to be limited to any specific needle park embodiment.

Referring now to FIG. 4 there is shown a plan view, similar to FIG. 2, of an alternate embodiment of a suture package 200 made in accordance with the present invention. In the embodiment illustrated in FIG. 4, it can be seen that the slot 207 connecting the central dispensing port 206 with the periphery of the package 200 is relatively narrow. It is preferred that the slot 207 is narrower than the diameter of the suture material so that when a piece of suture material is passed through the slot 207 it will be retained by friction. As seen in the side elevation view of FIG. 5, it is preferred that the slot 207 is formed at an angle. The embodiment shown in FIG. 4 also has elongated openings 208 that permit greater variation in the placement of the pins used in the winding process described above. Finally, it should be noted that the peripheral openings and walls described above with reference to FIG. 2 have been eliminated.

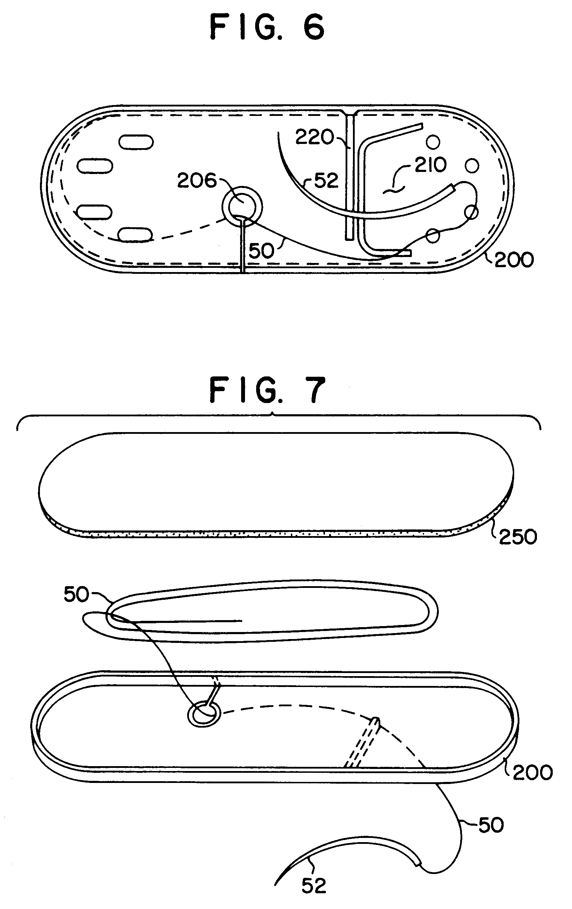

FIG. 6 is a plan view of the suture package shown in FIG. 4, loaded with a suture 50 and a needle 52. Preferably, the needle 52 is parked on the side of the package opposite the suture wind, seen in phantom. In one dispensing method, the needle 52 is grasped and pulled away from the package 200 and the suture material 50 is pulled through the central dispensing port 206. Since the suture 50 uncoils from the center of a spiral in the reverse of the direction in which it was wound, there is essentially no twisting or knotting. Alternatively, the package can be dispensed by pushing out the desiccant sheet (described below) using a needle holder so that the suture material 50 falls away from the package and uncoils. The latter dispensing method is known as "self-dispensing" since it will occur as a natural result of the needle holder pressing against a flap 210 located in the vicinity of the needle park 220.

FIG. 7 is an exploded perspective view of the loaded suture package 200 shown in FIG. 6, illustrating the placement of a sheet of desiccant material 250. The desiccant backing sheet 250, in addition to absorbing excess moisture, acts as an encapsulating structure to retain the suture material 50 in the configuration into which it has been wound. The types of materials that are preferably used as desiccant backing sheets include 12 'point' paper desiccant backing. In the case of "dry" synthetic products the package is loaded in a dry, evacuated and sterile atmosphere. However, gut products are loaded with alcohol and the desiccant is used to retain this fluid in the package.

The present invention provides a package with a number of significant advantages. The package described above is easily adaptable to any type of suture material and can accommodate any useful length of suture material since there is no defined winding channel of limited cross-section. The provision for readily accepting and discarding the sheet of desiccant material for forming the backing makes the package useful for absorbable sutures as well as any other type. The design of the needle park also permits any type of needle to be securely retained. These features and the central dispensing port permit a simple rotary winding that reduces shape retention or "memory" and also eliminates the likelihood of twisting. The winding as used in the package of the present invention also permits "self-dispensing," as described above. Finally, the disclosed design is compact and simple to mold, reducing cost and storage space requirements.

Although certain embodiments of the present invention have been described in detail, these embodiments are meant to describe the present invention, but do not limit its scope. Upon review of the foregoing description, those of skill in the art will realize numerous adaptation, modifications and alternate embodiments that utilize the concepts disclosed. Accordingly, reference should be made to the appended claims in order to ascertain the full scope of the present invention.

## Claims

1. A package (100;200) for retaining suture material, comprising a body (101;201) having two sides, a flexible flap (110;210) formed in the body (101;201), a sheet of desiccant material (120;250) and a peripheral structure (105;205); characterised in that the peripheral structure retains the sheet of desiccant material (120;250) in a position adjacent one side of the body (101;201), the sheet of desiccant material (120;250) retaining the suture material in a wound configuration between the sheet of desiccant material (120;250) and the one side of the body (101;201).

2. The package (100;200) of claim 1, further comprising a central dispensing port (106;206).

3. The package (100;200) of claim 1, further comprising a slot (107;207) connecting the central dispensing port (106;206) to a peripheral edge of the body (101;201).

4. The package (100;200) of claim 3, wherein the slot (107;207) is of a width less than a diameter of a suture material to be wound in the package (100;200).

5. The package (100;200) of claim 1, further comprising winding holes (108;208;209).

6. The package (100;200) of claim 1, wherein the peripheral structure (105;205) comprises a peripheral rim extending around at least a portion of the body (101;201).

7. The package (100;200) of claim 6, wherein the peripheral rim (105;205) extends completely around the body (101;201).

8. The package (100;200) of claim 1, wherein the peripheral structure (105;205) extends above one side of the body (101;201).

9. The package (100;200) of claim 1, wherein the peripheral structure (105;205) extends above the two sides of the body (101;201).

10. The package (100;200) of claim 1, further comprising one or more peripheral openings (112) disposed adjacent the peripheral structure (105;205).

## Patentansprüche

1. Verpackung (100, 200) zum Halten von Nahtmaterial aus einem Grundkörper (101, 201) mit zwei Seiten, mit einer im Grundkörper (101, 201) ausgebildeten flexiblen Klappe (110, 210), einem Blatt aus trocknendem Material (120, 250) und einer Umfangsanordnung (105, 205), dadurch gekennzeichnet, daß die Umfangsanordnung (105, 205) das Blatt aus trocknendem Material (120, 250) in einer Position angrenzend an eine Seite des Grundkörpers (101, 201) hält und das Blatt aus trocknendem Material (120, 250) das Nahtmaterial in gewickelter Anordnung zwischen dem Blatt aus trocknendem Material (120, 250) und einer Seite des Grundkörper (101, 201) hält.

2. Verpackung (100, 200) nach Anspruch 1, welche weiterhin eine zentrale Abgabeöffnung (106, 206) aufweist.

3. Verpackung (100, 200) nach Anspruch 1, welche weiterhin einen Schlitz (107, 207) aufweist, welcher die zentrale Abgabeöffnung (106, 206) mit einer Umfangskante des Grundkörpers (101, 201) verbindet.

4. Verpackung (100, 200) nach Anspruch 3, bei welcher der Schlitz (107, 207) eine Breite hat, die geringer als der Durchmesser des in der Verpackung (100, 200) aufzuwickelnden Nahtmaterials ist.

5. Verpackung (100, 200) nach Anspruch 1, welche weiterhin Wickellöcher (108, 208, 209) aufweist.

6. Verpackung (100, 200) nach Anspruch 1, bei welcher die Umfangsanordnung (105, 205) aus einer Umfangskante besteht, die sich um mindestens einen Teil des Grundkörpers (101, 201) erstreckt.

7. Verpackung (100, 200) nach Anspruch 6, bei welcher sich die Umfangskante (105, 205) vollständig rund um den Grundkörper (101, 201) erstreckt.

8. Verpackung (100, 200) nach Anspruch 1, bei welcher sich die Umfangsanordnung (105, 205) über eine Seite des Grundkörpers (101, 201) erstreckt.

9. Verpackung (100, 200) nach Anspruch 1, bei welcher sich die Umfangsanordnung (105, 205) über zwei Seiten des Grundkörpers (101, 201) erstreckt.

10. Verpackung (100, 200) nach Anspruch 1, welche weiterhin eine oder mehrere Umfangsöffnungen (112) aufweist, die angrenzend an die Umfangsanordnung (105, 205) angeordnet sind.

## Revendications

1. Emballage (100 ; 200) pour retenir un fil à suture comprenant un corps (101 ; 201) ayant deux côtés, un volet flexible (110 ; 210) formé dans le corps (101 ; 201), une feuille de matière asséchante (120 ; 250) et une structure périphérique (105 ; 205),
caractérisé
en ce que la structure périphérique retient la feuille de matière asséchante (120 ; 250) dans une position adjacente à un côté du corps (101 ; 201), la feuille de matière asséchante (120 ; 250) retenant le fil à suture dans une configuration enroulée entre la feuille de matière asséchante (120 ; 250) et le côté du corps (101 ; 201).

2. Emballage (100 ; 200) selon la revendication 1, comprenant en outre un orifice distributeur central (106 ; 206).

3. Emballage (100 ; 200) selon la revendication 1, comprenant en outre une fente (107 ; 207) reliant l'orifice distributeur central (106 ; 206) à un bord périphérique du corps (101 ; 201).

4. Emballage (100 ; 200) selon la revendication 3, dans lequel la fente (107 ; 207) est d'une largeur inférieure au diamètre d'un fil à suture à enrouler dans l'emballage (100 ; 200).

5. Emballage (100 ; 200) selon la revendication 1, comprenant en outre des trous d'enroulement (108 ; 208 ; 209).

6. Emballage (100 ; 200) selon la revendication 1, dans lequel la structure périphérique (105 ; 205) comprend un rebord s'étendant autour d'au moins une partie du corps (101 ; 201).

7. Emballage (100 ; 200) selon la revendication 6, dans lequel le rebord périphérique (105 ; 205) s'étend complètement autour du corps (101 ; 201).

8. Emballage (100 ; 200) selon la revendication 1, dans lequel la structure périphérique (105 ; 205) s'étend au-dessus d'un côté du corps (101 ; 201).

9. Emballage (100 ; 200) selon la revendication 1, dans lequel la structure périphérique (105 ; 205) s'étend au-dessus des deux côtés du corps (101 ; 201).

10. Emballage (100 ; 200) selon la revendication 1, comprenant en outre une ou plusieurs ouvertures périphériques (112) disposées en étant adjacentes à la structure périphérique (105 ; 205).
